# EUROPEAN PATENT APPLICATION

(11) **EP 4 813 370 A1**
(43) Date of publication of application: **30.09.2026**
(21) Application number: 24894527.1
(22) Date of filing: 15.11.2024
(51) Int. Cl.: A61K 8/92, A61K 8/37, A61K 8/891, A61K 8/86, A61K 8/39, A61K 8/03, A61Q 5/10, A61Q 5/12

(54) **OXIDATIVE HAIR DYE COMPOSITION**

(30) Priority: 20.11.2023 KR 20230160631
(71) Applicant: UCL Co. Ltd., Jeju-si, Jeju-do 63038 (KR)
(72) Inventor: OK, Hye Joo, Incheon 21367 (KR); KANG, Hye Su, Gunpo-si, Gyeonggi-do 15821 (KR); BAEK, Seok Yun, Seongnam-si, Gyeonggi-do 13611 (KR); LEE, Ji Won, Seoul 06276 (KR); PARK, Jin Oh, Seoul 06276 (KR)
(74) Representative: Eisenführ Speiser
(86) International application number: PCT/KR2024/018060
(87) International publication number: WO 2025/110634

(57) **Abstract**

The present invention relates to an oxidizing hair dye composition comprising: a first agent comprising an oil and an oxidizing dye; and a second agent comprising an oxidizing agent; and a hair dye comprising the oxidizing hair dye composition. The hair dye composition of the present invention contains an oil in the first agent to prevent contact between the oxidizing dye and oxygen, thereby inhibiting oxidation and providing excellent color retention, conditioning, and scalp protection effects.

## Description

### [TECHNICAL FIELD]

The present invention relates to an oxidizing hair dye composition comprising: a first agent comprising an oil and an oxidizing dye; and a second agent comprising an oxidizing agent; and a hair dye comprising the oxidizing hair dye composition.

### [BACKGROUND ART]

With the recent improvement in living standards, interest in beauty is increasing, and hair dyeing, which allows for the expression of individuality, has also become popularized.

Among hair dyes for dyeing hair, an oxidizing hair dye composition typically comprises a first agent comprising one or more oxidizing dye intermediates and one or more couplers, and dyeing is performed by mixing the first agent with a second agent comprising an oxidizing agent consisting of a hydrogen peroxide solution and the like, immediately before application to the hair. The purpose of using such hair dyes is to color the hair as completely as possible and to maintain the dyeing effect for a long time.

Conventional hair dye compositions are generally formed as a single formulation such as a gel, a cream, or a liquid formulation; however, cream formulations are inconvenient to use as they require a separate tool such as a brush to be applied evenly to the hair, while liquid formulations are inconvenient to use as the liquid drips onto the skin or contaminates the surrounding area during dyeing.

An oxidizing hair dye composition is typically dyed by mixing a first agent comprising one or more oxidizing dye precursors, one or more couplers, an alkali agent, an antioxidant, and a reducing agent, and a second agent comprising hydrogen peroxide or the like, immediately before a treatment, and applying the mixture to the hair. However, the first agent contains an oxidizing dye, so it oxidizes quickly, and the alkali agent included in the first agent and the hydrogen peroxide in the second agent cause scalp irritation and hair damage. Research is actively underway to address these issues; for example, methods utilizing silicones and cationic polymers to enhance hair conditioning effects have been studied. However, silicones pose a risk of harm to the environment and biodiversity and can accumulate on the scalp, potentially compromising scalp health. Furthermore, cationic polymers can also cause scalp itching and scalp irritation. Consequently, there is an urgent need to develop a new hair dye that can maintain the dyeing effect while preventing hair damage.

Korean Patent Laid-Open Publication No. 10-2020-0011674 discloses a hair dye additive composition containing coconut flower sugar, shrimp powder, and kelp powder, but a configuration for preventing self-oxidation of the first agent is not separately disclosed.

Against this background, the present inventors have made diligent research efforts to develop a hair dye capable of conditioning hair, preventing damage, and protecting the scalp. As a result, the present inventors have completed the present invention by confirming that when a hair dye composition is prepared with a first agent formed in a two-layer phase including an oil, oxidation is inhibited, and scalp irritation is alleviated when dyeing with the hair dye composition.

### [SUMMARY OF THE INVENTION]

### [TECHNICAL PROBLEM]

The present invention aims to solve the above-mentioned problems and other problems associated therewith.

One exemplary object of the present invention is to provide an oxidizing hair dye composition comprising: a first agent comprising an oil and an oxidizing dye; and a second agent comprising an oxidizing agent.

Another exemplary object of the present invention is to provide a hair dye comprising the oxidizing hair dye composition.

The technical problems to be achieved according to the technical spirit of the invention disclosed herein are not limited to the problems mentioned above, and other problems not mentioned will be clearly understood by those skilled in the art from the following description.

### [TECHNICAL SOLUTION]

This will be described in detail as follows. Meanwhile, each description and embodiment disclosed in this application may be applied to other descriptions and embodiments. That is, all combinations of the various elements disclosed in this application fall within the scope of this application. In addition, the scope of this application cannot be construed as being limited by the specific descriptions described below.

As one aspect for achieving the above object, the present invention provides an oxidizing hair dye composition comprising: a first agent comprising an oil and an oxidizing dye; and a second agent comprising an oxidizing agent.

In the present invention, the first agent may be formed of a plurality of layers comprising an upper layer comprising the oil, and a lower layer formed below the upper layer and comprising the oxidizing dye.

In one embodiment of the present invention, the first agent may be formed in two layers comprising an upper layer comprising the oil and a lower layer of an aqueous phase comprising the oxidizing dye.

Since an oxidizing dye included in a conventional hair dye is easily oxidized in the air, an antioxidant and/or a reducing agent are essentially included to prevent this. In addition, since the conventional hair dye requires filling with nitrogen when filling the dye into a container, the process is not only complicated but also disadvantageous in terms of cost. On the other hand, the present invention places an oil on the upper part of the aqueous phase of the first agent, so that the oil prevents contact between oxygen and the oxidizing dye to inhibit oxidation, and a separate nitrogen filling process is unnecessary, thereby making the manufacturing process simpler and providing excellent economic efficiency.

In the present invention, the oil may be at least one selected from the group consisting of an ester oil, a hydrocarbon oil, a silicone oil, and a vegetable oil.

In the present invention, the ester oil may be cetyl ethylhexanoate, dicaprylyl carbonate, isopropyl myristate, isopropyl palmitate, caprylic/capric triglyceride, triethylhexanoin, coco-caprylate/caprate, isoamyl laurate, C12-15 alkyl benzoate, tripentanoin, neopentanoate, neopentyl glycol diheptanoate, PEG-7 glyceryl cocoate, PEG-7 caprylic/capric glycerides, ethylhexyl palmitate, cetyl palmitate, dicaprylyl carbonate, or cetyl ethylhexanoate.

In the present invention, the hydrocarbon oil may be mineral oil, squalane, hydrogenated polydecene, undecane, tridecane, polybutene, hydrogenated polybutene, or C₁₃₋₁₅ alkane.

In the present invention, the silicone oil may be dimethicone, cyclopentasiloxane, cyclohexasiloxane, diphenylsiloxy phenyl trimethicone, phenyl trimethicone, or caprylyl methicone.

In the present invention, the vegetable oil may be sweet almond oil, corn oil, soybean oil, rapeseed oil, cottonseed oil, coconut oil, apricot kernel oil, castor oil, meadowfoam seed oil, grape seed oil, macadamia seed oil, olive oil, sunflower seed oil, jojoba seed oil, argan kernel oil, avocado oil, green tea seed oil, evening primrose oil, camellia seed oil, baobab seed oil, citron seed oil, flaxseed oil, or Brazil nut seed oil.

In the present invention, the oil may be included in an amount of 5 parts by weight or more, specifically 8 parts by weight or more, and more specifically 10 parts by weight or more, based on 100 parts by weight of the first agent in total.

In one embodiment of the present invention, the oil may be included in an amount of 5 to less than 30 parts by weight, specifically 5 to 25 parts by weight, and more specifically 5 to 20 parts by weight, based on 100 parts by weight of the first agent in total.

If the oil is less than 5 parts by weight based on 100 parts by weight of the first agent in total, contact with air cannot be completely blocked, making it impossible to prevent oxidation, and if it is 30 parts by weight or more, the proportion of the oil is excessively high, which may result in a very slow layer separation speed of the first agent.

Specifically, in an example of the present invention, when the first agent containing 30% or 40% of the oil content was left for 24 hours, layer separation was not smoothly performed, whereas when the oil was contained at less than 30%, it was confirmed that layer separation was smoothly performed.

In the present invention, the first agent may comprise a surfactant.

In the present invention, the HLB (Hydrophile-Lipophile Balance) value of the surfactant may be 10 to 30, specifically 10 to 25, and more specifically 10 to 21.

Specifically, in an example of the present invention, when the HLB value of the surfactant included in the first agent was 10 or more, the layer separation of the first agent was well performed at an appropriate speed, but when the HLB value was 3.7, 4.9, or 8, it was confirmed that the layer separation was slow or the water-soluble dye was decomposed and the phase ratio appeared to be heterogeneous.

In the present invention, the surfactant may be included in an amount of 0.1 to 1.5 parts by weight, and specifically 0.3 to 1.5 parts by weight, based on 100 parts by weight of the first agent in total.

Specifically, in an example of the present invention, when the first agent contained 0.3%, 0.8%, 0.5%, and 1% of the surfactant, respectively, layer separation was well performed; however, when the surfactant was not included, an issue where the interface was uneven and the surface was not smooth appeared, and when 2% was included, a result in which layer separation did not occur was confirmed.

In the present invention, the surfactant may be at least one selected from an anionic surfactant, a nonionic surfactant, a cationic surfactant, and an amphoteric surfactant.

In the present invention, the anionic surfactant may be at least one selected from sodium laureth sulfate, sodium lauryl sulfate, ammonium laureth sulfate, ammonium lauryl sulfate, MIPA-laureth sulfate, potassium cocoyl glycinate, disodium laureth sulfosuccinate, sodium cocoyl isethionate, sodium C₁₄₋₁₆ olefin sulfonate, sodium cocoyl glycinate, sodium lauroyl glutamate, sodium cocoyl alaninate, sodium methyl cocoyl taurate, and sodium lauroyl methylaminopropionate.

In the present invention, the nonionic surfactant may be at least one selected from sorbitan sesquioleate, coceth-7, PPG-1-PEG-9 lauryl glycol ether, PEG-40 hydrogenated castor oil, PEG-60 hydrogenated castor oil, polysorbate 20, polysorbate 60, polysorbate 80, octyldodeceth-5, octyldodeceth-25, decyl glucoside, oleth-2, oleth-5, and oleth-10.

In the present invention, the cationic surfactant may be at least one selected from cetrimonium chloride, laurtrimonium chloride, behentrimonium chloride, dicocodimonium chloride, cetrimonium methosulfate, behentrimonium methosulfate, and steartrimonium chloride.

In the present invention, the amphoteric surfactant may be at least one selected from coco-betaine, disodium cocoamphodiacetate, cocamidopropyl betaine, lauryl betaine, cocamidopropyl hydroxysultaine, and cocamide MIPA.

In the present invention, the oxidizing dye may be at least one selected from p-nitro-o-phenylenediamine, 2-amino-4-nitrophenol, 2-amino-5-nitrophenol, 2-amino-3-hydroxypyridine, 5-amino-o-cresol, m-aminophenol, o-aminophenol, p-aminophenol, 2,4-diaminophenoxyethanol hydrochloride, toluene-2,5-diamine hydrochloride, m-phenylenediamine hydrochloride, p-phenylenediamine hydrochloride, hydroxypropylbis(N-hydroxyethyl-p-phenylenediamine) hydrochloride, toluene-2,5-diamine, p-phenylenediamine, N-phenyl-p-phenylenediamine, picramic acid, p-nitro-o-phenylenediamine sulfate, p-methylaminophenol sulfate, 5-amino-o-cresol sulfate, m-aminophenol sulfate, o-aminophenol sulfate, p-aminophenol sulfate, toluene-2,5-diamine sulfate, m-phenylenediamine sulfate, p-phenylenediamine sulfate, N,N-bis(2-hydroxyethyl)-p-phenylenediamine sulfate, 2,6-diaminopyridine, 2,4-diaminophenol hydrochloride, 1,5-dihydroxynaphthalene, sodium picramate, 2-amino-5-nitrophenol sulfate, o-chloro-p-phenylenediamine sulfate, 1-hydroxyethyl-4,5-diaminopyrazole sulfate, hydroxybenzomorpholine, 6-hydroxyindole, α-naphthol, resorcinol, 2-methylresorcinol, gallic acid, catechol, and pyrogallol.

In the present invention, the oxidizing dye may be 0.001 to 5 parts by weight based on 100 parts by weight of the first agent in total.

In the present invention, the first agent may further comprise a coupler.

The coupler may be at least one selected from 2-methyl-5-hydroxyethylaminophenol, p-amino-o-cresol, m-aminophenol, 2,4-diaminophenoxyethanol hydrochloride, m-phenylenediamine hydrochloride, m-phenylenediamine, N-methyl-p-phenylenediamine, p-amino-o-cresol sulfate, 4-ethoxym-phenylenediamine sulfate, m-phenylenediamine sulfate, α-naphthol, resorcinol, and 2-methylresorcinol.

In the present invention, the coupler may be 0.001 to 5 parts by weight based on 100 parts by weight of the first agent in total.

In the present invention, the first agent may further comprise an alkali agent.

The alkali agent may be at least one selected from ammonia water, monoethanolamine, triethanolamine, arginine, aminomethylpropanol, sodium hydroxide, potassium hydroxide, ammonium bicarbonate, sodium bicarbonate, and sodium carbonate.

In the present invention, the alkali agent may be 0.01 to 20 parts by weight based on 100 parts by weight of the first agent in total.

In the present invention, the first agent may further comprise a metal ion chelating agent, an antioxidant, and/or a conditioning agent.

In the present invention, the metal ion chelating agent may be at least one selected from EDTA, EDTA-2NA, EDTA-3NA, EDTA-4NA, tetrasodium etidronate, etidronic acid, pentasodium pentetate, sodium stannate, and tetrasodium pyrophosphate.

In the present invention, the metal ion chelating agent may be 0.01 to 1 part by weight based on 100 parts by weight of the first agent in total.

In the present invention, the antioxidant may be at least one selected from ascorbic acid, erythorbic acid, sodium sulfite, cysteine, cysteine hydrochloride, thioglycolate, and thioglycolic acid.

In the present invention, the antioxidant may be 0.01 to 2 parts by weight based on 100 parts by weight of the first agent in total.

In the present invention, the conditioning agent may be at least one selected from cellulose-based thickeners, cationic polymers, and polyols.

In the present invention, the second agent may further comprise a solvent, a thickener, a pH adjuster, an oxidizing agent, a conditioning agent, and/or a metal ion chelating agent.

In the present invention, the thickener may be at least one selected from carbomer, acrylates/beheneth-25 methacrylate copolymer, acrylates/ceteth-20 itaconate copolymer, and acrylates/C₁₀-₃₀ alkyl acrylate crosspolymer.

In the present invention, the thickener may be 1 to 10 parts by weight based on 100 parts by weight of the second agent in total.

In the present invention, the pH adjuster may be at least one selected from disodium phosphate, phosphoric acid, and salicylic acid.

In the present invention, the pH adjuster may be 0.01 to 5 parts by weight based on 100 parts by weight of the second agent in total.

In the present invention, the oxidizing agent may be hydrogen peroxide, but is not limited thereto.

In the present invention, the oxidizing agent may be 2.5 to 12 parts by weight based on 100 parts by weight of the second agent in total.

In the present invention, the conditioning agent may be at least one selected from cellulose-based thickeners, cationic polymers, and polyols.

In the present invention, the metal ion chelating agent may be at least one selected from EDTA, EDTA-2NA, EDTA-3NA, EDTA-4NA, tetrasodium etidronate, etidronic acid, pentasodium pentetate, sodium stannate, and tetrasodium pyrophosphate.

In the present invention, the metal ion chelating agent may be 0.01 to 1 part by weight based on 100 parts by weight of the second agent in total.

As another aspect for achieving the above object, the present invention provides a hair dye comprising the oxidizing hair dye composition.

In the present invention, the hair dye may be at least one formulation selected from the group consisting of a cream, a gel, a liquid, a powder, an aerosol, and an emulsion.

### [ADVANTAGEOUS EFFECTS OF THE INVENTION]

The hair dye composition of the present invention contains an oil in the first agent to prevent contact between the oxidizing dye and oxygen, thereby inhibiting oxidation and providing excellent color retention, conditioning, and scalp protection effects.

### [BRIEF DESCRIPTION OF DRAWINGS]

FIG. 1 shows a result of confirming layer separation according to the presence or absence of an oil by visually observing the first agent of the present invention (Comparative Example 3 and Example 1) after opening and 24 hours and 7 days have elapsed after preparation.
FIG. 2 shows a result of confirming a layer separation speed according to an HLB value of a surfactant by visually observing the first agent of the present invention (Comparative Examples 4 to 6, Example 10, Example 12, and Example 13) after 0 hours, 0.5 hours, and 12 hours have elapsed after preparation.
FIG. 3 shows a result of confirming a layer separation speed according to a surfactant content by visually observing the first agent of the present invention (Comparative Example 1, Comparative Example 2, Example 1, Example 8, Example 9, and Example 10) after 24 hours have elapsed after preparation.
FIG. 4 shows a result of confirming a layer separation speed according to a type of oil by visually observing the first agent of the present invention (Comparative Example 3, Example 1, Example 2, Example 3, and Example 11) after 24 hours have elapsed after preparation.
FIG. 5 shows a result of confirming a layer separation speed according to an oil content by visually observing the first agent of the present invention (Comparative Example 3, Example 1, and Examples 4 to 7) after 24 hours have elapsed after preparation.
FIG. 6 shows a result of confirming a scalp protection effect of a hair dye composition prepared by including each of the first agent of the present invention (Comparative Example 3 and Example 1).

### [MODES FOR CARRYING OUT THE INVENTION]

Hereinafter, the present invention will be described in more detail through the following examples. However, these examples are for illustrative purposes only, and the scope of the present invention is not limited to these examples alone.

### Example 1: Preparation of Hair Dye Composition

### 1-1. Preparation of First Agent

A first agent containing an oil was prepared with the composition ratios of Tables 1 and 2 below (Examples 1 to 13). As a surfactant, PEG-40 hydrogenated castor oil (coceth-7, PPG-1-PEG-9 lauryl glycol ether, PEG-40 hydrogenated castor oil), cetrimonium chloride, sodium laureth sulfate, disodium cocoamphodiacetate, sorbitan sesquioleate, or oleth-2 was applied, and as an oil, mineral oil, cetyl ethylhexanoate, sweet almond oil, or dimethicone was applied.

**[Table 1]**

| No | Ingredient name | Example 1 | Example 2 | Example 3 | Example 4 | Example 5 | Example 6 | Example 7 |
|---|---|---|---|---|---|---|---|---|
| 1 | Purified water | 78.300 | 78.300 | 78.300 | 83.3000 | 68.300 | 58.300 | 48.300 |
| 2 | Hydroxyethyl cellulose | 0.400 | 0.400 | 0.400 | 0.4000 | 0.400 | 0.400 | 0.400 |
| 3 | Polyquatemium-7 | 1.000 | 1.000 | 1.000 | 1.0000 | 1.000 | 1.000 | 1.000 |
| 4 | Propylene glycol | 2.000 | 2.000 | 2.000 | 2.0000 | 2.000 | 2.000 | 2.000 |
| 5 | Disodium EDTA | 0.100 | 0.100 | 0.100 | 0.1000 | 0.100 | 0.100 | 0.100 |
| 6 | Ascorbic acid | 0.500 | 0.500 | 0.500 | 0.5000 | 0.500 | 0.500 | 0.500 |
| 7 | Sodium sulfite | 0.300 | 0.300 | 0.300 | 0.3000 | 0.300 | 0.300 | 0.300 |
| 8 | Sodium chloride | 0.500 | 0.500 | 0.500 | 0.5000 | 0.500 | 0.500 | 0.500 |
| 9 | Colorant | 1.200 | 1.200 | 1.200 | 1.200 | 1.200 | 1.200 | 1.200 |
| 10 | Fragrance | 0.200 | 0.200 | 0.200 | 0.2000 | 0.200 | 0.200 | 0.200 |
| 11 | PEG-40 hydrogenated castor oil | 0.500 | 0.500 | 0.500 | 0.5000 | 0.500 | 0.500 | 0.500 |
| 12 | Cetrimonium chloride | | | | | | | |
| 13 | Sodium laureth sulfate | | | | | | | |
| 14 | Disodium cocoamphodiacetate | | | | | | | |
| 15 | Sorbitan sesquioleate | | | | | | | |
| 16 | Oleth-2 | | | | | | | |
| 17 | Ethanolamine | 5.0000 | 5.0000 | 5.0000 | 5.0000 | 5.0000 | 5.0000 | 5.0000 |
| 18 | Mineral oil | 10.000 | | | 5.000 | 20.000 | 30.000 | 40.000 |
| 19 | Cetyl ethylhexanoate | | 10.000 | | | | | |
| 20 | Sweet almond oil | | | 10.000 | | | | |
| 21 | Dimethicone | | | | | | | |

| | | | | | | | | |
|---|---|---|---|---|---|---|---|---|
| 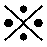 Expressed as weight % based on 100 parts by weight of the first agent in total | | | | | | | | |

**[Table 2]**

| No | Ingredient name | Example 8 | Example 9 | Example 10 | Example 11 | Example 12 | Example 13 |
|---|---|---|---|---|---|---|---|
| 1 | Purified water | 78.5000 | 78.0000 | 77.8000 | 78.300 | 77.8000 | 77.8000 |
| 2 | Hydroxyethyl cellulose | 0.4000 | 0.4000 | 0.4000 | 0.400 | 0.4000 | 0.4000 |
| 3 | Polyquatemium-7 | 1.0000 | 1.0000 | 1.0000 | 1.000 | 1.0000 | 1.0000 |
| 4 | Propylene glycol | 2.0000 | 2.0000 | 2.0000 | 2.000 | 2.0000 | 2.0000 |
| 5 | Disodium EDTA | 0.1000 | 0.1000 | 0.1000 | 0.100 | 0.1000 | 0.1000 |
| 6 | Ascorbic acid | 0.5000 | 0.5000 | 0.5000 | 0.500 | 0.5000 | 0.5000 |
| 7 | Sodium sulfite | 0.3000 | 0.3000 | 0.3000 | 0.300 | 0.3000 | 0.3000 |
| 8 | Sodium chloride | 0.5000 | 0.5000 | 0.5000 | 0.500 | 0.5000 | 0.5000 |
| 9 | Colorant | 1.200 | 1.200 | 1.200 | 1.200 | 1.200 | 1.200 |
| 10 | Fragrance | 0.2000 | 0.2000 | 0.2000 | 0.200 | 0.2000 | 0.2000 |
| 11 | PEG-40 hydrogenated castor oil | 0.3000 | 0.8000 | 1.0000 | 0.500 | | |
| 12 | Cetrimonium chloride | | | | | 1.000 | |
| 13 | Sodium laureth sulfate | | | | | | 1.000 |
| 14 | Disodium cocoamphodiacetate | | | | | | |
| 15 | Sorbitan sesquioleate | | | | | | |
| 16 | Oleth-2 | | | | | | |
| 17 | Ethanolamine | 5.0000 | 5.0000 | 5.0000 | 5.0000 | 5.0000 | 5.0000 |
| 18 | Mineral oil | 10.000 | 10.000 | 10.000 | | 10.000 | 10.000 |
| 19 | Cetyl ethylhexanoate | | | | | | |
| 20 | Sweet almond oil | | | | | | |
| 21 | Dimethicone | | | | 10.000 | | |

| | | | | | | | |
|---|---|---|---|---|---|---|---|
| 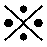 Expressed as weight % based on 100 parts by weight of the first agent in total | | | | | | | |

In addition, Comparative Examples 1 to 6 were prepared with different composition ratios as shown in Table 3 below.

**[Table 3]**

| No | Ingredient name | Comparative Example 1 | Comparative Example 2 | Comparative Example 3 | Comparative Example 4 | Comparative Example 5 | Comparative Example 6 |
|---|---|---|---|---|---|---|---|
| 1 | Purified water | 78.8000 | 76.800 | 88.300 | 77.8000 | 77.8000 | 77.8000 |
| 2 | Hydroxyethyl cellulose | 0.4000 | 0.400 | 0.400 | 0.4000 | 0.4000 | 0.4000 |
| 3 | Polyquaternium-7 | 1.0000 | 1.000 | 1.0000 | 1.0000 | 1.0000 | 1.0000 |
| 4 | Propylene glycol | 2.0000 | 2.000 | 2.000 | 2.0000 | 2.0000 | 2.0000 |
| 5 | Disodium EDTA | 0.1000 | 0.100 | 0.100 | 0.1000 | 0.1000 | 0.1000 |
| 6 | Ascorbic acid | 0.5000 | 0.500 | 0.500 | 0.5000 | 0.5000 | 0.5000 |
| 7 | Sodium sulfite | 0.3000 | 0.300 | 0.300 | 0.3000 | 0.3000 | 0.3000 |
| 8 | Sodium chloride | 0.5000 | 0.500 | 0.500 | 0.5000 | 0.5000 | 0.5000 |
| 9 | Colorant | 1.200 | 1.200 | 1.200 | 1.200 | 1.200 | 1.200 |
| 10 | Fragrance | 0.2000 | 0.200 | 0.200 | 0.2000 | 0.2000 | 0.2000 |
| 11 | PEG-40 hydrogenated castor oil | 0.0000 | 2.000 | 0.500 | | | |
| 12 | Cetrimonium chloride | | | | | | |
| 13 | Sodium laureth sulfate | | | | | | |
| 14 | Disodium cocoamphodiacetate | | | | | | 1.000 |
| 15 | Sorbitan sesquioleate | | | | 1.000 | | |
| 16 | Oleth-2 | | | | | 1.000 | |
| 17 | Ethanolamine | 5.0000 | 5.0000 | 5.0000 | 5.0000 | 5.0000 | 5.0000 |
| 18 | Mineral oil | 10.000 | 10.000 | | 10.000 | 10.000 | 10.000 |
| 19 | Cetyl ethylhexanoate | | | | | | |
| 20 | Sweet almond oil | | | | | | |
| 21 | Dimethicone | | | | | | |

| | | | | | | | |
|---|---|---|---|---|---|---|---|
| 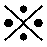 Expressed as weight % based on 100 parts by weight of the first agent in total | | | | | | | |

### 1-2. Preparation of second agent

A second agent was prepared with the composition ratios of Table 4 below (Examples 14 to 16). Specifically, after xanthan gum, carbomer, acrylates/ceteth-20 itaconate copolymer, and acrylates/C10-30 alkyl acrylate crosspolymer were added to purified water and stirred with an Agi mixer, propylene glycol, disodium EDTA, etidronic acid, disodium phosphate, and phosphoric acid were added and heated to 78°C to dissolve, then cooled to 40°C, and hydrogen peroxide was added and stirred to prepare the second agent.

**[Table 4]**

| No. | Ingredient name | Content (%) | | |
|---|---|---|---|---|
| | | Example 14 | Example 15 | Example 16 |
| 1 | Purified water | 90.930 | 91.730 | 90.730 |
| 2 | Xanthan gum | 0.100 | 0.100 | 0.100 |
| 3 | Carbomer | 1.800 | - | - |
| 4 | Acrylates/ceteth-20 itaconate copolymer | - | 1.000 | - |
| 5 | Acrylates/C10-30 alkyl acrylate crosspolymer | - | - | 2.000 |
| 6 | Propylene glycol | 1.000 | 1.000 | 1.000 |
| 7 | Disodium EDTA | 0.050 | 0.050 | 0.050 |
| 8 | Etidronic acid | 0.120 | 0.120 | 0.120 |
| 9 | Disodium phosphate | 0.350 | 0.350 | 0.350 |
| 10 | Phosphoric acid | 0.050 | 0.050 | 0.050 |
| 11 | Hydrogen peroxide | 5.600 | 5.600 | 5.600 |

| | | | | |
|---|---|---|---|---|
| 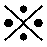 Expressed as weight % based on 100 parts by weight of the second agent in total | | | | |

### 1-3. Preparation of hair dye composition

After mixing the first agent (Example 1) and the second agent (Examples 14 to 16) in a 1:1 weight ratio, a viscosity was measured using an LVDV-II+Pro Brookfield Viscometer (64 pin, 12 rpm, 1 min), and the results are shown in Table 5.

**[Table 5]**

| Example | First agent | Example 1 | Example 1 | Example 1 |
|---|---|---|---|---|
| | Second agent | Example 14 | Example 15 | Example 16 |
| Viscosity (rpm) | | 21,895 | 25,447 | 22,593 |
| Appearance | | No flowability | No flowability | No flowability |

In addition, the first agent (Examples 1 to 13) and the second agent (Examples 14 to 16) were mixed in different combinations at a weight ratio of 1:1, respectively, to prepare an oxidizing hair dye composition, which was used for the evaluation of dyeing retention, conditioning, hair-nourishing, and scalp protection effects described below.

### Example 2: Evaluation of Oxidizing Power and Degree of Layer Separation of First Agent

### 2-1. Evaluation of Oxidizing Power

The first agent compositions of Example 1 and Comparative Example 3 were left open at room temperature for 24 hours or one week, respectively, and then the degree of color was confirmed visually. Example 1 contains a mineral oil, and Comparative Example 3 is a first agent composition containing no oil.

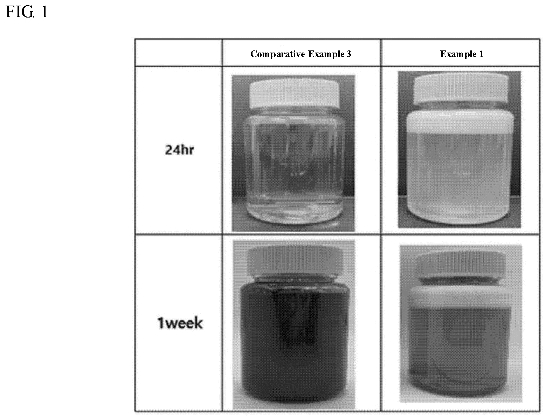

As a result, it was confirmed that the color of Comparative Example 3 became darker compared to Example 1 after one week had elapsed (FIG. 1), suggesting that the oil layer included in the composition of Example 1 is located on the upper part of the aqueous phase containing the dye to block oxygen in the air, thereby preventing oxidation of the dye. That is, the two-layer hair dye composition comprising the oil phase (oil layer) on the upper part of the aqueous phase has an oxidation inhibitory effect on the dye even without filling with nitrogen, and thus was evaluated as having not only long-term storage stability but also economic utility due to the unnecessity of packaging.

### 2-2. Evaluation of Layer Separation Speed According to HLB Value of Surfactant

By applying different surfactants, HLB (Hydrophile-Lipophile Balance,
hydrophilic-lipophilic balance) values were different, and Comparative Examples 4 to 6, Example 10, Example 12, and Example 13 were left at room temperature, and layer separation was visually observed by time (0 hours, 0.5 hours, and 12 hours).

The layer separation speed according to the difference in the HLB value was compared, and as for a separation time suitable for mass production, it is suitable that the separation is gradually performed after about 1 hour elapses after preparation, and is completely separated and maintained after 12 hours.

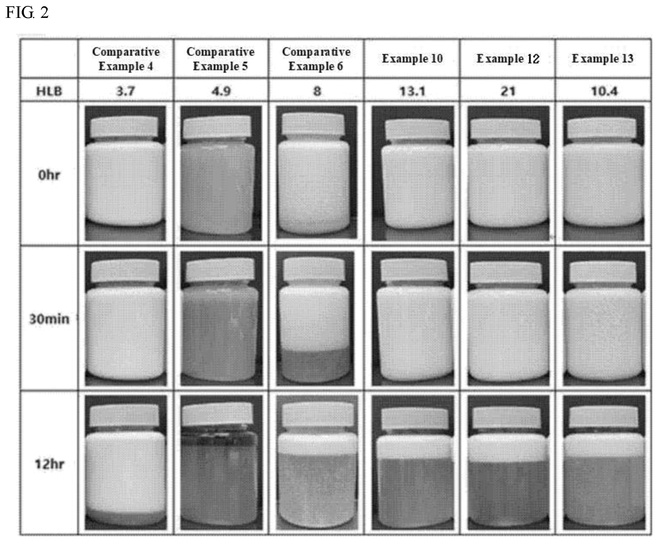

As a result, in the case of Comparative Example 4 in which sorbitan sesquioleate (HLB 3.7) was applied as the surfactant, the layer separation speed was too slow, and in oleth-2 (Comparative Example 5 in which HLB 4.9 was applied), the water-soluble dye was decomposed while being emulsified in the oil layer. In the case of Comparative Example 6 in which disodium cocoamphodiacetate (HLB 8) was applied, the layer separation started rapidly from 0.5 hours before leaving, indicating that the layer ratio between products may be heterogeneous when the products are filled. On the other hand, Example 10 in which PEG-40 hydrogenated castor oil (HLB 13.1) was applied as the surfactant, Example 12 in which cetrimonium chloride (HLB 21) was applied, and Example 13 in which sodium laureth sulfate (HLB 10.4) was applied showed an appropriate separation speed (FIG. 2).

### 2-3. Evaluation of Layer Separation Speed According to Surfactant Content

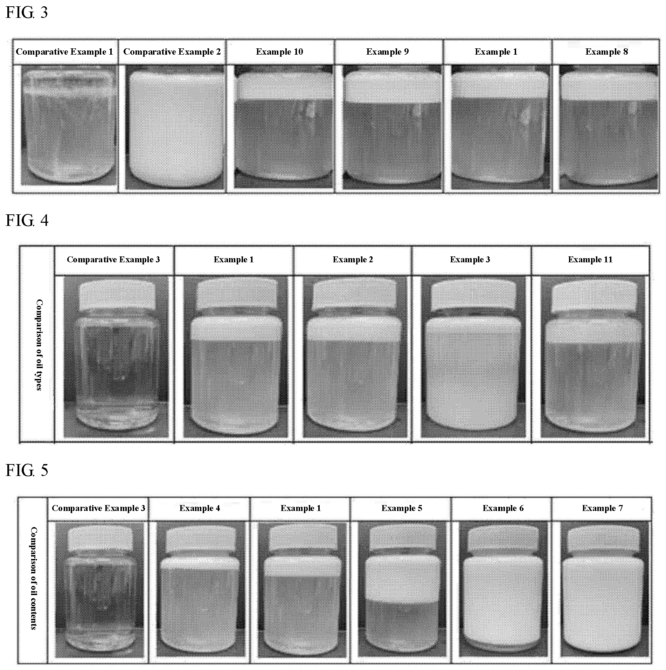

The effect of the content of the surfactant included in the first agent on layer separation was confirmed. As a result of comparing layer separation by leaving Comparative Example 1 containing i) no surfactant, Comparative Example 2 containing ii) 2% of the surfactant, Example 1 containing iii) 0.5% of the surfactant, Example 8 containing iv) 0.3% of the surfactant, Example 9 containing v) 0.8% of the surfactant, and Example 10 containing vi) 1% of the surfactant for 24 hours, it was confirmed that separation was well performed in Example 1, Example 8, Example 9, and Example 10. On the other hand, Comparative Example 1 not containing the surfactant immediately underwent layer separation and had an uneven interface, so that the surface was not smooth, and Comparative Example 2 containing 2% of the surfactant showed a result in which layer separation hardly occurred (FIG. 3).

### 2-4. Evaluation of Layer Separation Speed According to Type and Content of Oil

Comparative Example 3, Example 1 (mineral oil applied), Example 2 (cetyl ethylhexanoate applied), Example 3 (sweet almond oil applied), and Example 11 (dimethicone applied) having different types of included oils, respectively, were left at room temperature for 24 hours, and then the degree of separation was visually observed.

As a result, layer separation did not occur in Comparative Example 3 containing no oil, and it was confirmed that layer separation was achieved in Example 1, Example 2, Example 3, and Example 11. However, in the case of Example 3, the polarity of the oil was somewhat low, so it was confirmed that the oil was partially emulsified in the aqueous phase part and appeared cloudy (FIG. 4).

In addition, Comparative Example 3, Example 4 (oil content of 5%), Example 1 (oil content of 10%), Example 5 (oil content of 20%), Example 6 (oil content of 30%), and Example 7 (oil content of 40%) having different oil contents, respectively, were left at room temperature for 24 hours, and then the degree of separation was compared.

As a result, in the case of Example 6 and Example 7 having a high oil content, it was found that layer separation was not well performed (FIG. 5).

### Example 3: Evaluation of Dyeing Retention and Hair Protection Effect of Oxidizing Hair Dye Composition

### 3-1. Evaluation of Color Fastness (Dyeing Retention)

The hair dye composition prepared using Comparative Example 3 containing no oil as the first agent, and the hair dye compositions prepared using Examples 1 to 7 and Example 11 containing the oil as the first agent were each mixed with the second agent prepared in Example 14 at a ratio of 1:1, evenly applied to an experimental hair sample, left for 30 minutes, and then washed in running water using a shampoo liquid. Thereafter, color was measured using a spectrophotometer (Konica Minolta CM-5) on the hair dried using a hair dryer and the hair shampooed repeatedly 5 times to evaluate dyeing retention (color fastness).

*As the + value of a increases, it is closer to red, and as the - value increases, it is closer to green. As the + value of b increases, it is closer to yellow, and as the - value increases, it is closer to blue. The ΔE*ab value is a standard deviation value measuring a difference from a standard color, and indicates that the larger the value is, the larger the change in color is, and the smaller the value is, the smaller the change in color is.

The color fastness was compared using the ΔE*ab value measured by the spectrophotometer (Konica Minolta CM-5), the hair washed once and dried was set as a standard value, and the ΔE*ab value of the hair shampooed 5 times was measured a total of 3 times to obtain an average ΔE*ab value.

The smaller the ΔE*ab value, the smaller the change in color, indicating excellent dyeing retention.

As a result of comparing color fastness, it was confirmed that the dyeing retention of Examples 1 to 7 and Example 11 was remarkably excellent (Table 6).

**[Table 6]**

| | Comparative Example 3 | Example 1 | Example 2 | Example 3 | Example 4 | Example 5 | Example 6 | Example 7 | Example 11 |
|---|---|---|---|---|---|---|---|---|---|
| ΔE*ab (1 time) | 1.15 | 0.5 | 0.28 | 0.47 | 0.51 | 0.55 | 0.61 | 0.62 | 0.77 |
| ΔE*ab (2 times) | 1.1 | 0.75 | 0.29 | 0.73 | 0.7 | 0.37 | 0.59 | 0.2 | 0.31 |
| ΔE*ab (3 times) | 0.86 | 0.46 | 0.23 | 0.73 | 0.63 | 0.65 | 0.58 | 0.19 | 0.35 |
| ΔE*ab (Average) | 1.04 | 0.57 | 0.27 | 0.64 | 0.61 | 0.52 | 0.59 | 0.34 | 0.48 |

### 3-2. Evaluation of Conditioning Effect

The conditioning effect of the hair dye composition prepared by respectively applying the first agent of Comparative Example 3, Examples 1 to 7, and Example 11 was measured. That is, a friction force of a test hair having a weight of 1.5 g and a length of 100 mm was measured using a friction coefficient tester (Neo-Tribo(FCMS 170)), and each of the hair dye compositions containing Comparative Example 3, Examples 1 to 7, and Example 11 was evenly applied to the same hair. After drying the hair, the friction force was measured again using the friction coefficient tester (Neo-Tribo(FCMS 170)). A smaller average value of the friction coefficient means that the conditioning effect of the hair is more excellent.

As a result, as shown in Table 7, the friction coefficient values of Examples 1 to 7 and Example 11 containing an oil were lower than those of Comparative Example 3 containing no oil, confirming that the hair dye composition exhibited a superior conditioning effect when it contained an oil compared to when it did not.

**[Table 7]**

| | Comparative Example 3 | Example 1 | Example 2 | Example 3 | Example 4 | Example 5 | Example 6 | Example 7 | Example 11 |
|---|---|---|---|---|---|---|---|---|---|
| 1 | 0.269 | 0.224 | 0.176 | 0.156 | 0.231 | 0.226 | 0.202 | 0.123 | 0.142 |
| 2 | 0.252 | 0.224 | 0.174 | 0.156 | 0.229 | 0.230 | 0.199 | 0.129 | 0.135 |
| 3 | 0.251 | 0.227 | 0.175 | 0.158 | 0.228 | 0.217 | 0.202 | 0.129 | 0.136 |
| 4 | 0.246 | 0.290 | 0.171 | 0.154 | 0.232 | 0.216 | 0.197 | 0.128 | 0.131 |
| 5 | 0.242 | 0.230 | 0.168 | 0.154 | 0.228 | 0.217 | 0.195 | 0.129 | 0.132 |
| 6 | 0.241 | 0.227 | 0.167 | 0.153 | 0.228 | 0.219 | 0.191 | 0.126 | 0.131 |
| 7 | 0.240 | 0.227 | 0.168 | 0.149 | 0.227 | 0.219 | 0.190 | 0.124 | 0.128 |
| 8 | 0.242 | 0.231 | 0.166 | 0.147 | 0.231 | 0.216 | 0.190 | 0.126 | 0.126 |
| 9 | 0.243 | 0.230 | 0.169 | 0.146 | 0.230 | 0.209 | 0.188 | 0.125 | 0.127 |
| 10 | 0.241 | 0.231 | 0.169 | 0.149 | 0.230 | 0.205 | 0.181 | 0.123 | 0.121 |
| Average | 0.247 | 0.234 | 0.170 | 0.152 | 0.229 | 0.218 | 0.194 | 0.126 | 0.131 |

### 3-3. Evaluation of Hair-Nourishing Effect

The hair-nourishing effect when applying the first agent of Comparative Example 3, Example 1, Examples 3 to 5, and Example 7 was evaluated. The hair-nourishing effect refers to a measure by which hair can grow thick and healthy. After the hair dye composition prepared for each experimental group was applied to a hair swatch and dried, a gloss band appearing as light scattering on the surface of the hair was photographed under constant illumination, and then analyzed and evaluated. From the photographed image, the gloss band was extracted using an analysis program, and a pixel value of the area occupied by the gloss band relative to the total area was obtained. It indicates that as the area of the gloss band increases, the gloss increases.

As a result, as shown in Table 8, it was confirmed that the area of the gloss band significantly increased in Examples 1, 3, 5, and 7 containing 10 to 40% of the oil, compared to Comparative Example 3 containing no oil. However, in the case of Example 4 having an oil content of 5%, an area value similar to that of Comparative Example 3 was exhibited.

**[Table 8]**

| | Comparative Example 3 | Example 1 | Example 3 | Example 4 | Example 5 | Example 7 |
|---|---|---|---|---|---|---|
| Area Value (pixel) | 37,500 | 50,549 | 50,442 | 37,110 | 46,325 | 42,423 |

### 3-4. Evaluation of Scalp Protection Effect

For 10 adult males and females having no skin disease, 20 µl of each of the hair dye composition to which Comparative Example 3 was applied and the hair dye composition to which Example 1 was applied was closely attached to the skin of an arm region, and then fixed with an ECODERM IQ Ultra Patch and applied for 8 hours.

Visual evaluation by an examiner was performed according to the following criteria (Table 9) reflecting Frosch & Kligman and The Cosmetic, Toiletry, and Fragrance Association (CTFA) guidelines ( * 4 subjects were excluded from the experiment due to contact dermatitis at a patch contact site).

**[Table 9]**

| **Symbol** | **Score** | **Judgment Criteria** |
|---|---|---|
| + | 1 | Slight erythema, spots, or diffusion |
| ++ | 2 | Moderate uniform erythema |
| +++ | 3 | Severe erythema accompanied by edema |
| ++++ | 4 | Severe erythema accompanied by edema and blisters |

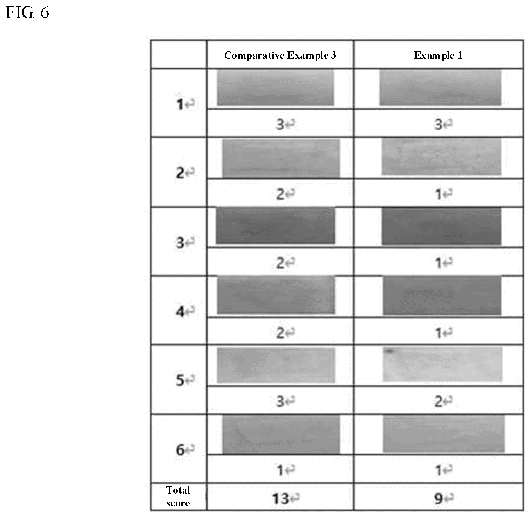

As a result, it was confirmed that when the first agent containing an oil (Example 1) was applied, the irritation value was smaller than that of the first agent containing no oil (Comparative Example 3), which was effective in alleviating skin irritation (FIG. 6). From the above description, those skilled in the art to which the present invention pertains will understand that the present invention can be carried out in other specific forms without changing the technical spirit or essential features thereof. In this regard, the embodiments described above should be understood as illustrative in all respects and not limiting. The scope of the present invention should be construed as being indicated by the meaning and scope of the claims to be described later rather than the detailed description, and all changes or modified forms derived from the equivalent concept thereof are included in the scope of the present invention.

## Claims

1. An oxidizing hair dye composition comprising:
a first agent comprising an oil and an oxidizing dye; and
a second agent comprising an oxidizing agent

2. The oxidizing hair dye composition of claim 1, wherein
the first agent is formed of a plurality of layers comprising an upper layer comprising the oil and a lower layer formed below the upper layer and comprising the oxidizing dye.

3. The oxidizing hair dye composition of claim 1, wherein
the oil is at least one selected from the group consisting of an ester oil, a hydrocarbon oil, a silicone oil, and a vegetable oil.

4. The oxidizing hair dye composition of claim 1, wherein
the oil is included in an amount of 5 parts by weight to less than 30 parts by weight based on 100 parts by weight of the first agent in total.

5. The oxidizing hair dye composition of claim 1, wherein
the first agent comprises a surfactant.

6. The oxidizing hair dye composition of claim 5, wherein
an HLB (Hydrophile-Lipophile Balance) value of the surfactant is 10 to 30.

7. The oxidizing hair dye composition of claim 5, wherein
the surfactant is included in an amount of 0.1 to 1.5 parts by weight based on 100 parts by weight of the first agent in total.

8. A hair dye comprising the oxidizing hair dye composition of claim 1.

9. The hair dye of claim 8, wherein
the hair dye is at least one formulation selected from the group consisting of a cream, a gel, a liquid, a powder, an aerosol, and an emulsion.
